# EUROPEAN PATENT SPECIFICATION

(11) **EP 3 560 490 B1**
(45) Date of publication and mention of the grant of the patent: **06.11.2024**
(21) Application number: 17883760.5
(22) Date of filing: 22.12.2017
(51) Int. Cl.: A61K 31/192, A61K 31/704, A61K 9/16, A61K 9/20, A61K 9/28, A61P 29/00

(54) **ORAL IBUPROFEN PREPARATION**
ORALES IBUPROFEN-PRÄPARAT
PRÉPARATION D'IBUPROFÈNE POUR LA VOIE ORALE

(30) Priority: 22.12.2016 JP 2016249261
(43) Date of publication of application: 30.10.2019
(73) Proprietor: SSP Co., Ltd., Japan, Tokyo 163-1488 (JP)
(72) Inventor: YAMAGUCHI Tomihisa, Tokyo 163-1488 (JP); UMINO Makoto, Narita-shi Chiba 286-8511 (JP); MIYADAI Nobuo, Tokyo 163-1488 (JP)
(74) Representative: Santarelli
(86) International application number: PCT/JP2017/046056
(87) International publication number: WO 2018/117249

(56) References cited:
- EP-A1- 0 465 235
- JP-A- 2000 264 838
- JP-A- 2007 023 026
- JP-A- 2014 070 024
- JP-A- H04 230 330
- JP-A- H06 199 676
- JP-A- S63 198 620
- OKABE S ET AL: "Effects of Fm100, Fm 100-DeG and glycyrrhizin on gastric secretion and experimental gastriculcers in rats", OYO YAKURI - PHARMACOMETRICS, OYO YAKURI KENKYUKAI, JP, vol. 18, no. 3, 1 January 1979 (1979-01-01), pages 469 - 474, XP009521469, ISSN: 0300-8533
- DEHPOUR A. R. ET AL.: "Antiulcer activities of liquorice and its derivatives in experimental gastric lesion induced by ibuprofen in rats", INTERNATIONAL JOURNAL OF PHARMACEUTICS, vol. 119, no. 2, 1 January 1995 (1995-01-01), pages 133 - 138, XP055612299, DOI: 10.1016/0378-5173(94)00377-H

## Description

### [Technical Field]

The present invention relates to an oral ibuprofen preparation, and relates more specifically to an oral ibuprofen preparation comprising ibuprofen, magnesium oxide and glycyrrhizic acid, whereby gastric mucosal damage caused by ibuprofen is greatly reduced.

### [Background Art]

Non-steroidal antipyretic and analgesic anti-inflammatory agents are used to alleviate fever and pain, and medicines containing ibuprofen in particular are widely prescribed and marketed. Although ibuprofen has excellent anti-inflammatory and analgesic effects, it is also known to have the drawback of causing gastric mucosal damage.

Research has previously been done into eliminating such drawbacks of ibuprofen, and for example, techniques are known for using ibuprofen in combination with magnesium carbonate and the like (PTL 1), using ibuprofen in combination with magnesium oxide (PTL 2), and using ibuprofen in combination with caffeine (NPL 1) and the like.

However, although the gastric mucosa-damaging effects of ibuprofen have been ameliorated to some degree by the techniques described above and the like, formulations such as over-the-counter formulations for example that consumers can use at their own discretion are needed to ensure greater safety, and there is a need for the development of techniques capable of suppressing gastric mucosal damage more effectively than in the past.

### [Citation List]

### [Patent Literature]

[PTL 1] Japanese Patent No. 2704721
[PTL 2] Japanese Patent Application Publication No. 2007-23026
[PTL 3] Japanese Patent No. 5959393
European Patent Application No. 0465235 discloses a composition comprising ibuprofen and magnesium oxide.

### [Non Patent Literature]

[NPL 1] J. Pharm. Pharmacol. Vol. 51 (1999), p. 817-824

### [Summary of Invention]

### [Technical Problem]

Consequently, it is an object of the present invention to provide an oral ibuprofen preparation whereby the gastric mucosa-damaging effects of ibuprofen are improved at a high level.

### [Solution to Problem]

Looking at known oral ibuprofen preparations that combine ibuprofen with antacids such as magnesium oxide, the inventors focused on the reducing effects of these preparations on gastric mucosal damage, and after exhaustive research into means of increasing these effects, we perfected the present invention after discovering that the gastric mucosa-damaging effects could be further reduced by including glycyrrhizic acid, which is a component of licorice.

That is, the present invention is an oral ibuprofen preparation containing ibuprofen, magnesium oxide, and glycyrrhizic acid as defined in the appended claims.

The present invention is this oral ibuprofen preparation wherein the magnesium oxide content is 0.1 to 2.0 parts by weight per 1 part by weight of ibuprofen, and this oral ibuprofen preparation wherein the glycyrrhizic acid content is 0.01 to 0.2 parts by weight per 1 part by weight of ibuprofen.

### [Advantageous Effects of Invention]

The oral ibuprofen preparation of the invention can be widely used as a highly safe anti-inflammatory and analgesic agent, cold medicine and the like because it is unlikely to cause gastric mucosal damage when ingested orally.

### [Brief Description of Drawings]

[Fig. 1]

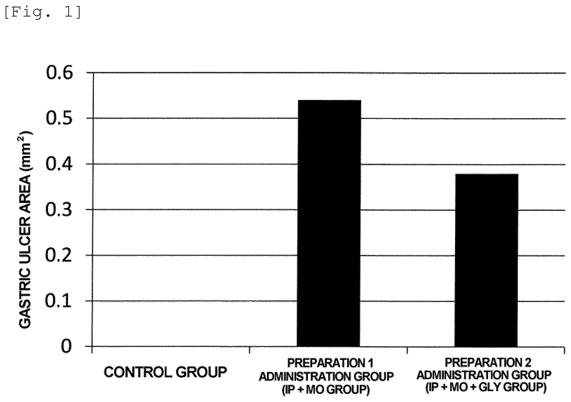

Fig. 1 is a histogram showing gastric ulcer area in Test Example 1.

### [Description of Embodiments]

The oral ibuprofen preparation of the invention contains ibuprofen, magnesium oxide, and glycyrrhizic acid as essential components as defined in the appended claims.

Ibuprofen used in the invention is a well-known anti-inflammatory and analgesic component, and in addition to ibuprofen, pharmacologically acceptable salts of ibuprofen may also be used in the present invention.

The magnesium oxide used in the present invention is also a compound known as an antacid component, and is manufactured by oxidizing magnesium, normally by baking magnesium hydroxide at a high temperature. Magnesium oxides are classified into heavy magnesium oxide and light magnesium oxide, and heavy magnesium oxide with a specific volume of 2 to 4 mL/g or especially 3 to 4 mL/g is preferably used in the invention.

Glycyrrhizic acid is a triterpene glycoside contained in licorice, and is used as an artificial sweetener, and in external preparations and the like. Glycyrrhizic acid or a pharmacologically acceptable salt thereof, such as for example dipotassium glycyrrhizinate or the like, may be used as the glycyrrhizic acid. Glycyrrhizic acid is sometimes compounded in oral medicines with the hope of relieving inflammation of the throat mucosa and the like due to colds and the like, but suppression of gastric mucosal damage and the like has not been reported.

In preparing the oral ibuprofen preparation of the present invention (hereunder called the "preparation of the invention"), these essential components may be formulated appropriately in combination with any known pharmacologically acceptable components, but the following points need to be kept in mind when compounding.

First, the compounded ratio of ibuprofen and magnesium oxide is 0.1 to 2.0 parts by weight, or preferably 0.3 to 1.0 parts by weight of magnesium oxide per 1 part by weight of ibuprofen. This is because as shown in PTL 2 (especially Fig. 1) and Table 1 of PTL 3 (Comparative Example 4 and Comparative Example 13), when ibuprofen and magnesium are compounded together they may actually promote gastric mucosal damage if the proportions are not within a specific range.

Moreover, the compounded ratio of ibuprofen and glycyrrhizic acid is 0.01 to 0.2 parts by weight, or preferably 0.06 to 0.09 parts by weight of glycyrrhizic acid per 1 part by weight of ibuprofen. If the compounded ratio is below this range, a sufficient gastric mucosal damage suppression effect may not be obtained. On the other hand, no further effects may be obtained if the compounded ratio is above this range, and the proportions may also be unsuitable for formulation.

The preparation of the invention may be in the form of a tablet, granules, powder, capsules and appropriate formulation methods may be used depending on the form. That is, it is sufficient to mix these three essential components uniformly with other necessary components to obtain the desired preparation. When preparing a powder for example, the uniform mixture may be used as is as the preparation, while in the case of granules, a solvent such as water may be added as necessary to the uniform mixture, which can then be granulated by known methods. In the case of a tablet, the uniform mixture or granulated product can be tableted by ordinary methods, while in the case of a capsule, the uniform mixture or granulated product can be packed in a capsule by ordinary methods.

Examples of optional components that can be used when manufacturing the preparation of the invention include sugars such as lactose, sucrose, glucose, and mannitol as carriers; excipients such as crystalline cellulose; binders such as gelatin, sodium alginate, hydroxypropyl cellulose, hydroxypropyl methyl cellulose, polyvinylpyrrolidone, and carboxymethyl cellulose; disintegrants such as carboxymethyl cellulose calcium, low-substituted hydroxypropyl cellulose, and calcium carbonate; lubricants such as magnesium stearate, and talc; and hydrogenated vegetable oils such as hardened castor oil, and dissolution aids, buffers, preservatives, perfumes, dyes, flavorings and the like may also be used as necessary.

The preparation of the invention thus obtained can normally be made into a form that can be administered to an adult subject at a daily dose of ibuprofen of 90 to 1,200 mg, preferably 200 to 600 mg, and more preferably 450 to 600 mg in which the daily dose may be divided and administered 2 or 3 times per day, although the dose will differ depending on the purpose of use and age of a subject.

While adequately providing the anti-inflammatory and analgesic effects of the compounded ibuprofen, the preparation of the invention explained above can suppress the occurrence of gastric mucosal damage, or in other words gastric mucosal lesions such as erosion, bleeding and edemas of the stomach and the adjacent upper digestive tract including the duodenum through the action of the combined magnesium oxide and glycyrrhizic acid.

Consequently, the preparation of the invention can be widely used as a highly safe antipyretic, analgesic, migraine treatment, inflammation treatment, cold medicine or the like.

### [Examples]

The present invention is explained in more detail below based on test examples and examples.

### Test Example 1

### Gastric mucosal damage suppression effect:

The following preparations containing ibuprofen as an anti-inflammatory and analgesic component were prepared, and their effects on the gastric mucosa were studied in rats using gastric mucosal damage as an indicator.

Rats (male SD rats) that had shown no abnormalities in general condition observation during the quarantine and habituation period were used as experimental animals. Using the weight of the rats on the last day of quarantine as a indicator, the rats were divided into 3 groups of 10 rats each by the stratified continuous randomization method.

180 mg of ibuprofen (IP) and 120 mg of magnesium oxide (MO) were dissolved and dispersed in 100 mL of 0.5% CMC solution to prepare a Preparation 1, and the same amounts of ibuprofen and magnesium oxide were dissolved and dispersed in 100 mL of 0.5% CMC solution together with 15.6 mg of glycyrrhizic acid (GLY) to prepare a Preparation 2.

Each preparation was administered orally in an amount equal to 5 mL/kg based on body weight to a rat group that had fasted overnight since the day before administration and had had no water for 2 hours before administration, and these were called the Preparation 1 administration group and Preparation 2 administration group. A group receiving only 5% CMC solution in place of the preparations was also established as a control (control group).

4 hours after administration of each preparation, the rats were decapitated under isoflurane anesthesia, and the stomachs were extracted. 10 mL of 2% formalin solution was injected into each stomach, and the stomachs were then lightly fixed by being soaked with the solution for about 10 minutes. After being fixed, the stomachs were incised along the greater curvature, and photographed so that ulcers occurring in the glandular stomach were clearly evident. Ulcer area was calculated based on these photographs.

### <Results>

The gastric ulcer areas are shown in Fig. 1. As shown in the figure, the gastric ulcer area was 0.00 mm² (here and below, average value) in the control group, 0.54 mm² in the Preparation 1 administration group (IP + MO group), and 0.38 mm² in the Preparation 2 administration group (IP + MO + GLY group).

The usual gastric ulcer area when ibuprofen is administered alone in the same amount given to the Preparation 1 group is about 0.64 mm², so gastric mucosal damage was reduced by about 16% in the Preparation 1 administration group, in which the gastric ulcer area was 0.54 mm². Gastric mucosal damage was reduced by 41% in the Preparation 2 administration group, in which the gastric ulcer area was 0.38 mm². Thus, the rate of reduction in gastric mucosal damage caused by ibuprofen when magnesium oxide and glycyrrhizic acid were included was 260% (2.6 times) of that obtained when only magnesium oxide was compounded, showing clearly that an anti-inflammatory and analgesic agent causing little gastric mucosal damage could be obtained by including glycyrrhizic acid.

### Preparation Example 1

### Granules:

The various ingredients were compounded to obtain the following formulation per packet, mixed, and kneaded and dried by ordinary methods to manufacture granules.

### <Formulation>

| Ingredient | Compounded amount |
|---|---|
| Ibuprofen | 75 mg |
| Magnesium oxide | 50 mg |
| Glycyrrhizic acid | 6.5 mg |
| Corn starch | 66.5 mg |
| Magnesium stearate | 2 mg |
| Total | 200 mg |

### Preparation Example 2

### Tablet:

The various ingredients were compounded to obtain the following formulation per tablet, mixed, and tableted by ordinary methods to manufacture a tablet.

### <Formulation>

| Ingredient | Compounded amount |
|---|---|
| Ibuprofen | 75 mg |
| Magnesium oxide | 50 mg |
| Glycyrrhizic acid | 6.5 mg |
| Crystalline cellulose | 40 mg |
| Low-substituted hydroxypropyl cellulose | 25 mg |
| Light anhydrous silicic acid | 1.5 mg |
| Magnesium stearate | 1 mg |
| Talc | 1 mg |
| Total | 200 mg |

### Preparation Example 3

### Tablet:

The various ingredients were compounded to obtain the following formulation per tablet, mixed, and tableted by ordinary methods to manufacture a tablet. This uncoated tablet was coated with 15 mg of hydroxypropyl methylcellulose per tablet to manufacture a film-coated tablet.

### <Formulation>

| Ingredient | Compounded amount |
|---|---|
| Ibuprofen | 75 mg |
| Magnesium oxide | 50 mg |
| Glycyrrhizic acid | 6.5 mg |
| Crystalline cellulose | 20 mg |
| Low-substituted hydroxypropyl cellulose | 30 mg |
| Mannitol | 15 mg |
| Light anhydrous silicic acid | 1.5 mg |
| Magnesium stearate | 1 mg |
| Talc | 1 mg |
| Total | 200 mg |

### Preparation Example 4

### Tablet:

The various ingredients were compounded to obtain the following formulation per tablet, mixed, and tableted by ordinary methods to manufacture a tablet.

### <Formulation>

| Ingredient | Compounded amount |
|---|---|
| Ibuprofen | 100 mg |
| Magnesium oxide | 50 mg |
| Glycyrrhizic acid | 6.5 mg |
| Allyl propyl acetyl urea | 30 mg |
| Anhydrous caffeine | 40 mg |
| Crystalline cellulose | 60 mg |
| Low-substituted hydroxypropyl cellulose | 35 mg |
| Lactose | 17.5 mg |
| Hydroxypropyl cellulose | 5 mg |
| Light anhydrous silicic acid | 2 mg |
| Magnesium stearate | 4 mg |
| Total | 350 mg |

### Preparation Example 5

### Tablet:

The various ingredients were compounded to obtain the following formulation per tablet, mixed, and tableted by ordinary methods to manufacture a tablet. This uncoated tablet was coated with 15 mg of hydroxypropyl methylcellulose per tablet to manufacture a film-coated tablet.

### <Formulation>

| Ingredient | Compounded amount |
|---|---|
| Ibuprofen | 100 mg |
| Magnesium oxide | 50 mg |
| Glycyrrhizic acid | 6.5 mg |
| dl-methylephedrine hydrochloride | 10 mg |
| Isopropamide iodide | 1 mg |
| Anhydrous caffeine | 12.5 mg |
| Crystalline cellulose | 85 mg |
| Low-substituted hydroxypropyl cellulose | 45 mg |
| Corn starch | 77 mg |
| Hydroxypropyl cellulose | 4 mg |
| Light anhydrous silicic acid | 3 mg |
| Magnesium stearate | 3 mg |
| Talc | 3 mg |
| Total | 400 mg |

### Preparation Example 6

### Tablet:

The various ingredients were compounded to obtain the following formulation per tablet, mixed, and tableted by ordinary methods to manufacture a tablet. This uncoated tablet was coated with 15 mg of hydroxypropyl methylcellulose per tablet to manufacture a film-coated tablet.

### <Formulation>

| Ingredient | Compounded amount |
|---|---|
| Ibuprofen | 100 mg |
| Magnesium oxide | 75 mg |
| Glycyrrhizic acid | 6.5 mg |
| dl-methylephedrine hydrochloride | 10 mg |
| Isopropamide iodide | 1 mg |
| Anhydrous caffeine | 12.5 mg |
| Crystalline cellulose | 85 mg |
| Low-substituted hydroxypropyl cellulose | 45 mg |
| Corn starch | 77 mg |
| Hydroxypropyl cellulose | 4 mg |
| Light anhydrous silicic acid | 3 mg |
| Magnesium stearate | 3 mg |
| Talc | 3 mg |
| Total | 425 mg |

### Preparation Example 7

### Tablet:

The various ingredients were compounded to obtain the following formulation per tablet, mixed, and tableted by ordinary methods to manufacture a tablet. This uncoated tablet was coated with 20 mg of hydroxypropyl methylcellulose per tablet to manufacture a film-coated tablet.

### <Formulation>

| Ingredient | Compounded amount |
|---|---|
| Ibuprofen | 100 mg |
| Magnesium oxide | 100 mg |
| Glycyrrhizic acid | 6.5 mg |
| dl-methylephedrine hydrochloride | 10 mg |
| Isopropamide iodide | 1 mg |
| Anhydrous caffeine | 12.5 mg |
| Crystalline cellulose | 85 mg |
| Low-substituted hydroxypropyl cellulose | 45 mg |
| Corn starch | 77 mg |
| Hydroxypropyl cellulose | 4 mg |
| Light anhydrous silicic acid | 3 mg |
| Magnesium stearate | 3 mg |
| Talc | 3 mg |
| Total | 450 mg |

## Claims

1. An oral ibuprofen preparation comprising ibuprofen, magnesium oxide, and glycyrrhizic acid, wherein the content of the magnesium oxide is 0.1 to 2.0 parts and the content of the glycyrrhizic acid is 0.01 to 0.2 parts by weight per 1 part by weight of ibuprofen.

2. The oral ibuprofen preparation according to Claim 1, wherein the content of the magnesium oxide is 0.3 to 1.0 part by weight per 1 part by weight of ibuprofen.

3. The oral ibuprofen preparation according to Claim 1 or 2, wherein the content of the glycyrrhizic acid is 0.06 to 0.09 part by weight per 1 part by weight of ibuprofen.

4. The oral ibuprofen preparation according to any of Claims 1 to 3, wherein the preparation is for administering ibuprofen to an adult subject at a daily dose of 90 to 1,200 mg.

5. The oral ibuprofen preparation according to any of Claims 1 to 4, wherein the preparation is a tablet, granules, a powder, or a capsule.

## Patentansprüche

1. Orales Ibuprofen-Präparat, das Ibuprofen, Magnesiumoxid und Glycyrrhizinsäure umfasst, wobei der Gehalt an Magnesiumoxid 0,1 bis 2,0 Gewichtsteile und der Gehalt an Glycyrrhizinsäure 0,01 bis 0,2 Gewichtsteile pro 1 Gewichtsteil Ibuprofen beträgt.

2. Orales Ibuprofen-Präparat nach Anspruch 1, wobei der Gehalt an Magnesiumoxid 0,3 bis 1,0 Gewichtsteile pro 1 Gewichtsteil Ibuprofen beträgt.

3. Orales Ibuprofen-Präparat nach Anspruch 1 oder 2, wobei der Gehalt an Glycyrrhizinsäure 0,06 bis 0,09 Gewichtsteile pro 1 Gewichtsteil Ibuprofen beträgt.

4. Orales Ibuprofen-Präparat nach einem der Ansprüche 1 bis 3, wobei das Präparat zur Verabreichung von Ibuprofen an einen erwachsenen Patienten in einer täglichen Dosis von 90 bis 1.200 mg dient.

5. Orales Ibuprofen-Präparat nach einem der Ansprüche 1 bis 4, wobei das Präparat eine Tablette, Granula, ein Pulver oder eine Kapsel ist.

## Revendications

1. Préparation d'ibuprofène pour voie orale comprenant de l'ibuprofène, de l'oxyde de magnésium, et de l'acide glycyrrhizique, dans laquelle la teneur de l'oxyde de magnésium est de 0,1 à 2,0 parties et la teneur de l'acide glycyrrhizique est de 0,01 à 0,2 partie en poids pour 1 partie en poids d'ibuprofène.

2. Préparation d'ibuprofène pour voie orale selon la revendication 1, dans laquelle la teneur de l'oxyde de magnésium est de 0,3 à 1,0 partie en poids pour 1 partie en poids d'ibuprofène.

3. Préparation d'ibuprofène pour voie orale selon la revendication 1 ou 2, dans laquelle la teneur de l'acide glycyrrhizique est de 0,06 à 0,09 partie en poids pour 1 partie en poids d'ibuprofène.

4. Préparation d'ibuprofène pour voie orale selon l'une quelconque des revendications 1 à 3, dans laquelle la préparation est destinée à une administration d'ibuprofène à un sujet adulte à la posologie journalière de 90 à 1 200 mg.

5. Préparation d'ibuprofène pour voie orale selon l'une quelconque des revendications 1 à 4, dans laquelle la préparation est un comprimé, des granules, une poudre, ou une capsule.
